# EUROPEAN PATENT APPLICATION

(11) **EP 2 921 280 A1**
(43) Date of publication of application: **23.09.2015**
(21) Application number: 13855735.0
(22) Date of filing: 19.11.2013
(51) Int. Cl.: B29C 51/02, B29C 51/30, B29C 51/10

(54) **METHOD FOR THERMOFORMING FLEXIBLE SHEET PARTS**

(30) Priority: 19.11.2012 WO PCT/ES2012/070804
(71) Applicant: Simplicity Works Europe S.L., 03205 Elche (Alicante) (ES)
(72) Inventor: HERNÁNDEZ HERNÁNDEZ, Adrián, E-03670 Alicante (ES)
(74) Representative: Carvajal y Urquijo, Isabel
(86) International application number: PCT/ES2013/070799
(87) International publication number: WO 2014/076347

(57) **Abstract**

A method for thermoforming flexible sheet pieces via a tool having a deformable surface (1) made up of innumerable independent small areas (2), supported by axially mobile rods (3) and mounted in a sealed box (4). Gaps (6) communicating with the sealed chamber (4) are defined between the independent areas (2), through which a vacuum is applied on the lower surface of the sheet (7) to be formed.

## Description

### Field of invention

The present invention relates to a method for thermoforming flexible sheet pieces, which enables three-dimensional surfaces to be reproduced in order to obtain pieces or objects with finished shapes, especially plastic-based materials.

The method of the invention is carried out via a tool comprising at least one deformable surface upon which - once the shapes to be obtained have been reproduced - the sheet piece to be formed is abutted and the thermoforming thereof is carried out, by means of applying heat to the sheet piece, which is later pushed against the surface reproduced in the tool.

### Background of the invention

US6640413 makes known a method for thermoforming plastic sheet pieces using a mould made from a deformable surface. A sheet piece to be formed is arranged on the deformable surface, then the same is thermoformed by applying heat on the sheet piece, as a vacuum is meanwhile created between the sheet piece and the surface of the mould, through suction holes.

Another problem is that each time different shapes are formed, a new mould must be made.

US4510636 makes known a tool for forming shoe inserts, which is made up of innumerable abutting and axially mobile rods, which are mounted in a box. Via the application of certain shapes (sole of the foot) on the outer end of the rods, the same shapes are reproduced at the inner end. The piece to shape the shoe insert is applied onto the shapes reproduced at the inner end of the rods, which remain inside the box and is formed via pressurised air being pumped into the chamber. In this tool, the formed shoe insert is carried out on the end of the rods opposite the one on which the shape to be obtained was reproduced and furthermore, the forming is carried out via direct pressure being applied on the outer surface of the shoe insert, opposite the support surface on the rods.

### Description of the invention

The object of the present invention is a method for thermoforming flexible sheet pieces, of the variety initially stated, in which the surface reproduced on the deformable surface of the tool is provided with innumerable points and/or lines through which a vacuum is created, which are furthermore situated close to one another. Thus, when the sheet pieces to be formed are placed on the reproduced surface of the mould and heat and a vacuum is applied, a complete adaptation of the same to the shapes of the mould is achieved, along with a perfect reproduction of the desired shape in the sheet piece.

According to another aspect of the invention, thermoforming the sheet piece is carried out via a tool that has a deformable surface, made up of innumerable independent small areas, which may be supported by axially mobile rods or be made up of the cross-section of said rods, which are mounted in a sealed lower chamber.

In accordance with the invention, the independent small areas are separated by gaps that communicate with the sealed lower chamber, which in turn is provided with an opening through which said chamber may be connected with a vacuum source. With this arrangement, the necessary vacuum effect on the lower surface of the sheet piece to enable this piece to be applied on the shape reproduced by the ends of the rods and adapt perfectly to said shapes, may be applied through the aforementioned gaps.

The method of the invention is carried out with a mould of the variety presented and comprises the following stages:
a) Reproducing the shapes desired on the tool with the deformable surface;
b) Heating the flexible sheet piece until the optimum softening point has been reached;
c) Abutting the sheet piece to be shaped against the shapes reproduced;
d) Thermoforming said sheet piece by applying a vacuum.

In accordance with the invention, the deformable surface of the mould is made up of innumerable independent small areas separated by gaps through which the vacuum is applied to the sheet piece, in order to push said piece against the independent areas and keep them abutted against the same, all of which is carried out as heat is meanwhile being applied on the sheet piece.

As stated above, the tool with which the method of the invention is carried out comprises a deformable surface made up of innumerable independent small areas, supported by axially mobile rods or defined by the cross-section of the rods themselves, which are mounted in a sealed lower chamber. In accordance with the invention, the independent small areas that define the deformable surface are separated by gaps that communicate with the sealed chamber, which is provided with an opening so that it may be connected with a vacuum source. The gaps that separate the independent small areas may consist of holes, grooves or both holes and grooves.

The rods may be pushed towards the extended position by any system and may furthermore be provided with means for blocking said rods in any retracted position, which when freed from these means return to the extended position.

The movement of the independent areas may be achieved via pressure being applied to the same or by pulling the rods that support them. In the first case, the shape to be obtained with the tool may be achieved by resting and pressing the model to reproduce onto the set of independent areas, in order to achieve the movement of the same until the shapes of the surface resting thereon are reproduced. In the second case, the shapes to be obtained on the deformable surface of the tool may be achieved by pulling the rods that support the plates, for example, through a computer-controlled mechanical system.

### Brief description of the drawings

The method of the invention may be better understood in light of the accompanying drawings, wherein
- Figure 1 is a plan view of a tool for carrying out the method of the invention.
- Figure 2 shows a longitudinal cross-section of the tool in figure 1, with a foot applied on the same.
- Figure 3 corresponds to the detailed section A at a larger scale.
- Figure 4 is a similar view to that of figure 2, with a plastic material thermoformed on the tool.

### Detailed description of an embodiment

In the accompanying drawings a tool is shown comprising a platform (1) made up of a base of innumerable small-sized independent areas (2), which are all situated on the same plane and each one of which is supported by an axially mobile rod (3).

All the rods (3) are parallel and are mounted in a support box (4), such that they may be moved axially between a fully extended position, in which the areas (2) are in a coplanar position and a fully retracted position, which depends on the length of the rod (3). As previously stated, all the rods are axially mobile and are provided with means that secure and maintain the position thereof at any height and also enable then to return to the fully extended position.

The set of small areas (2) defines a deformable surface on which the mould to be reproduced is applied.

In the example shown in the drawings, the mould to be reproduced consists of the sole of a foot.

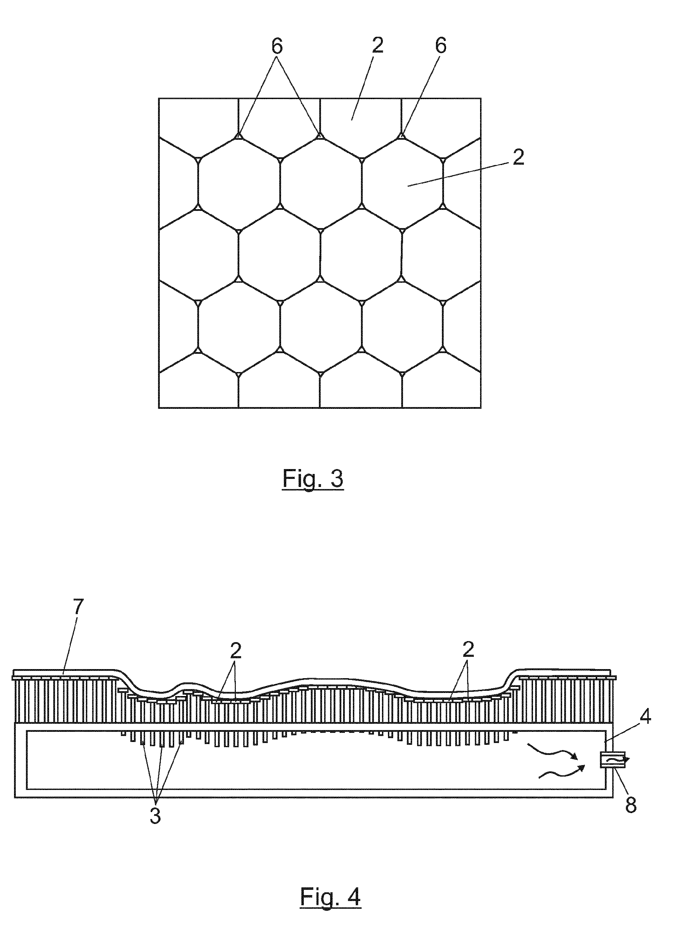

In order to do so, as shown in the drawings, a person's feet (5) are applied on the platform (1), pressing the sole against the deformable surface defined by the set of areas (2), which move to varying degrees, depending on the zone of the foot applied on the same. There are separation gaps, in the form of small holes (6) for example, between the consecutive areas or plates (2), as shown in figure 3.

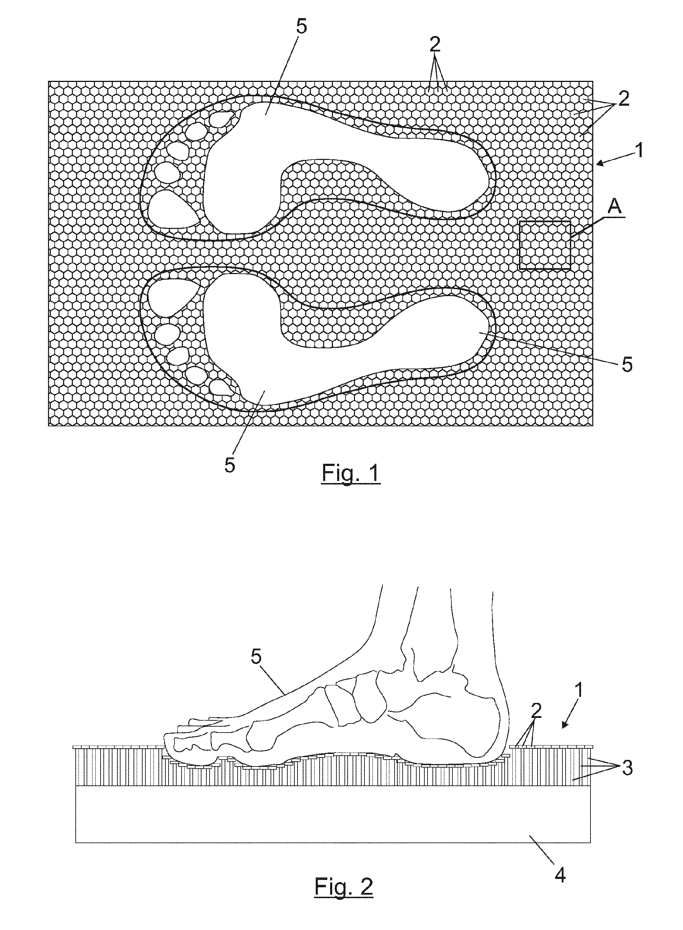

As shown in figure 2, the shape of the sole of the foot is reproduced on the platform (1), due to the difference in movement of the different areas (2). This reproduced shape is maintained in the tool when the foot (5) is removed, as shown in figure 4.

A thermoformable sheet (7) is subsequently arranged on the shapes reproduced on the platform (1), figure 4, to which heat is applied so as to be thermoformed, as said sheet is meanwhile pushed against the shapes of the platform (1). In order to do so, the chamber (4) is provided with an opening or nozzle (8) to which a vacuum source may be connected, which acts on the lower surface of the sheet (7) through the holes (6).

As such, the vacuum is applied to the lower surface of the sheet (7) through the holes (6), figure 3, thus causing it to adapt to the shapes reproduced by the movement of the areas (2). The heat needed to form the sheet (7) may be applied from the upper surface of said sheet.

With the make-up described, the tool from which the shape to be reproduced is obtained is made up of a surface formed by innumerable points, thus enabling the configuration of any body to be scanned in order to obtain a surface that may also be modified by varying the coordinates of the small areas (2), via the corresponding software for example.

In conclusion, moulds and items in general with different configurations may be manufactured with the method described from the deformable surface of the tool, which scans the shapes to be reproduced, later enabling the configuration of the points thereof to be changed, in order to introduce corrective concepts, all of which always guarantees perfect adaptation of the sheet piece to be formed on the surfaces reproduced in the tool.

The coordinates of the different areas (2), which depend on the position of the rods (3) supporting them, may be controlled via a computerised mechanical system, thus operating as a periphery of a computer that controls the position of each area (2) via the corresponding software.

The areas (2), as previously stated, may consist of or be defined by the transverse cross-section of the rods (3) themselves, between which the gaps through which the vacuum is applied to the lower surface of the sheet to be formed are defined.

As shown in figure 3, the independent small areas (2) may be hexagon shaped, thus abutting one another laterally. The gaps (6) through which the vacuum is applied to the sheet (7) to be formed consist of holes (6) that are situated at the point of coincidence of the vertices of adjacent independent areas (2).

## Claims

1. A method for thermoforming flexible sheet pieces, via a tool having a deformable surface made up of innumerable independent small areas, which may be moved in the same direction, the method of which comprises reproducing the shapes to be obtained on said deformable surface, applying heat to the sheet piece, abutting the sheet pieces to be formed against the shape reproduced and thermoforming said sheet piece via a vacuum, **characterised in that** the independent small areas are separated by gaps through which the vacuum is applied to the sheet piece, in order to push and abut it against the independent areas.

2. A tool for thermoforming flexible sheet pieces, comprising a deformable surface made up of innumerable independent small areas supported by axially mobile rods, which are mounted in a sealed chamber, **characterised in that** the independent small areas are separated by gaps that communicate with the sealed chamber and the sealed chamber is provided with an opening so that it may be connected to a vacuum source.

3. The tool, according to claim 2, **characterised in that** the independent small areas are hexagon shaped and abut laterally, the gaps being made up of holes situated at the point of coincidence of the vertices of adjacent areas.
